# EUROPEAN PATENT SPECIFICATION

(11) **EP 3 045 102 B1**
(45) Date of publication and mention of the grant of the patent: **03.04.2019**
(21) Application number: 14860055.4
(22) Date of filing: 26.12.2014
(51) Int. Cl.: A61B 1/018, A61B 18/14, A61B 1/273

(54) **MEDICAL SYSTEM**
MEDIZINISCHES SYSTEM
SYSTÈME MÉDICAL

(30) Priority: 08.11.2013 JP 2013232063
(43) Date of publication of application: 20.07.2016
(73) Proprietor: Olympus Corporation, Tokyo 192-8507 (JP)
(72) Inventor: MATSUI, Akira, Hachioji-shi, Tokyo 192-8507 (JP)
(74) Representative: Gunzelmann, Rainer
(86) International application number: PCT/JP2014/084652
(87) International publication number: WO 2015/068863

(56) References cited:
- EP-A1- 0 888 748
- WO-A1-2014/208106
- WO-A1-2014/208107
- JP-A- H1 199 158
- JP-A- S6 083 633
- JP-A- H11 128 241
- JP-A- 2000 271 137
- US-A- 6 106 519

## Description

### Technical Field

The present invention relates to a treatment device and a medical system, and particularly, to a treatment device and a medical system including the treatment device that is used by being inserted into a channel of an endoscope and that performs treatment and the like by utilizing wirelessly supplied power.

### Background Art

In a medical field, a tool, an apparatus, and the like for applying high-frequency current to living tissue to treat the living tissue are conventionally used.

More specifically, for example, high-frequency dissection forceps that are inserted into a body cavity while being inserted into a channel of an endoscope and that are configured to apply high-frequency current to living tissue to treat the living tissue are disclosed in U.S. Patent No. 7824407.

On the other hand, various tools and apparatuses corresponding to wireless power feeding used in the medical field are proposed in recent years.

More specifically, for example, configurations for wirelessly supplying power, through capacitive coupling, from a power transmission electrode provided on a trocar to a power reception electrode of a cordless surgical tool inserted into the trocar are disclosed in U.S. Patent No. 6187002 and U.S. Patent No. 6206875. Configurations including an urging mechanism that presses a power transmission electrode of a trocar against a power reception electrode of a cordless surgical tool inserted into the trocar are also disclosed in U.S. Patent No. 6187002 and U.S. Patent No. 6206875.

However, according to the configuration disclosed in U.S. Patent No. 7824407, a cable for supplying power necessary for operation, such as treatment, is connected to the high-frequency dissection forceps, and there is a problem that operability of the high-frequency dissection forceps is reduced.

According to the configurations disclosed in U.S. Patent No. 6187002 and U.S. Patent No. 6206875, the urging mechanism, such as a spring, presses the power transmission electrode against the power reception electrode of the surgical tool, and there is a problem that slidability of the surgical tool is reduced. Movement in forward and backward directions and rotation become difficult, and operability is reduced.

Document US 6 106 519 A refers to a surgical trocar including a closure tube of a capacitive electrosurgical instrument. The trocar includes a cannula housing, a cannula tube extending from the cannula housing, a trocar cannula and a capacitive electrosurgical adapter. The capacitive electrosurgical adapter is connected to the trocar cannula and includes a central aperture. The central aperture is an elongated aperture for receiving working instruments. The capacitive electrosurgical adapter further comprises a proximal capacitor plate and a distal capacitor plate. The proximal capacitor plate comprises a first proximal capacitor stator plate and a second proximal capacitor stator plate. The distal capacitor plate comprises a first distal capacitor stator plate and a second distal capacitor stator plate.

The present invention has been made in view of the circumstances, and an object of the present invention is to provide a treatment device and a medical system including the treatment device that can stabilize a supply state of power in wireless power feeding, while preventing a reduction in operability when the treatment device is used by inserting the treatment device into a channel of an endoscope.

### Disclosure of Invention

### Means for Solving the Problem

The invention is defined by the medical system of independent claim 1. Preferred embodiments are defined by the dependent claims. Any example or embodiment which does not fall under the scope of the independent claim is not part of the invention.

### Brief Description of the Drawings

Fig. 1 is a diagram showing a configuration of main parts of a medical system according to a first embodiment;
Fig. 2 is a cross-sectional schematic diagram of an endoscope of the medical system according to the first embodiment;
Fig. 3 is a cross-sectional schematic diagram of a treatment device according to the first embodiment;
Fig. 4 is a diagram showing an example of a configuration of protruding portions provided on the treatment device according to the first embodiment;
Fig. 5 is a cross-sectional view showing an example of a cross section cut along a line A-A';
Fig. 6 is a cross-sectional view showing an example of the cross section cut along the line A-A', the example different from Fig. 5;
Fig. 7 is a diagram showing an equivalent circuit of main parts of the medical system according to the first embodiment;
Fig. 8 is a diagram showing an example of the configuration of the protruding portion provided on the treatment device according to the first embodiment, the example different from Fig. 4;
Fig. 9 is a cross-sectional view showing a cross section cut along a line B-B';
Fig. 10 is a diagram showing an example of the configuration of the protruding portions provided on the treatment device according to the first embodiment, the example different from Figs. 4 and 8;
Fig. 11 is a cross-sectional view showing a cross section cut along a line C-C';
Fig. 12 is a diagram showing an example of the configuration of the protruding portions provided on the treatment device according to the first embodiment, the example different from Figs. 4, 8, and 10;
Fig. 13 is a cross-sectional view showing a cross section cut along a line D-D';
Fig. 14 is a diagram showing an example of the configuration of the protruding portion provided on the treatment device according to the first embodiment, the example different from Figs. 4, 8, 10, and 12;
Fig. 15 is a diagram showing an example of the configuration of the protruding portion provided on the treatment device according to the first embodiment, the example different from Figs. 4, 8, 10, 12, and 14;
Fig. 16 is a diagram showing an example of the configuration of the protruding portion provided on the treatment device according to the first embodiment, the example different from Figs. 4, 8, 10, 12, 14, and 15;
Fig. 17 is a schematic diagram showing an example in which part of an insertion portion of the treatment device in the medical system according to the first embodiment is deformed into an elliptic cylindrical shape;
Fig. 18 is a cross-sectional view showing a cross section cut along a line E-E';
Fig. 19 is a schematic diagram showing an example in which part of the insertion portion of the treatment device in the medical system according to the first embodiment is deformed into an elliptic cylindrical shape, the example different from Fig. 17;
Fig. 20 is a diagram showing a configuration of main parts of a medical system according to a second embodiment;
Fig. 21 is a cross-sectional schematic diagram of an endoscope of the medical system according to the second embodiment;
Fig. 22 is a cross-sectional schematic diagram of a treatment device according to the second embodiment;
Fig. 23 is a diagram showing an example of a configuration of protruding portions provided on the treatment device according to the second embodiment;
Fig. 24 is a cross-sectional view showing an example of a cross section cut along a line F-F';
Fig. 25 is a cross-sectional view showing an example of the cross section cut along the line F-F', the example different from Fig. 24; and
Fig. 26 is a diagram showing an equivalent circuit of main parts of the medical system according to the second embodiment.

### Best Mode for Carrying Out the Invention

Hereinafter, embodiments of the present invention will be described with reference to the drawings.

### (First Embodiment)

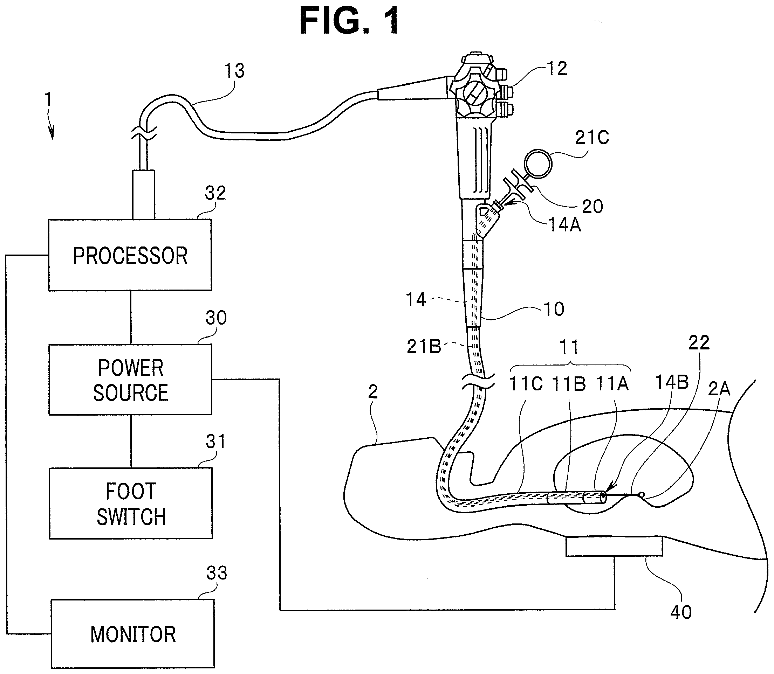

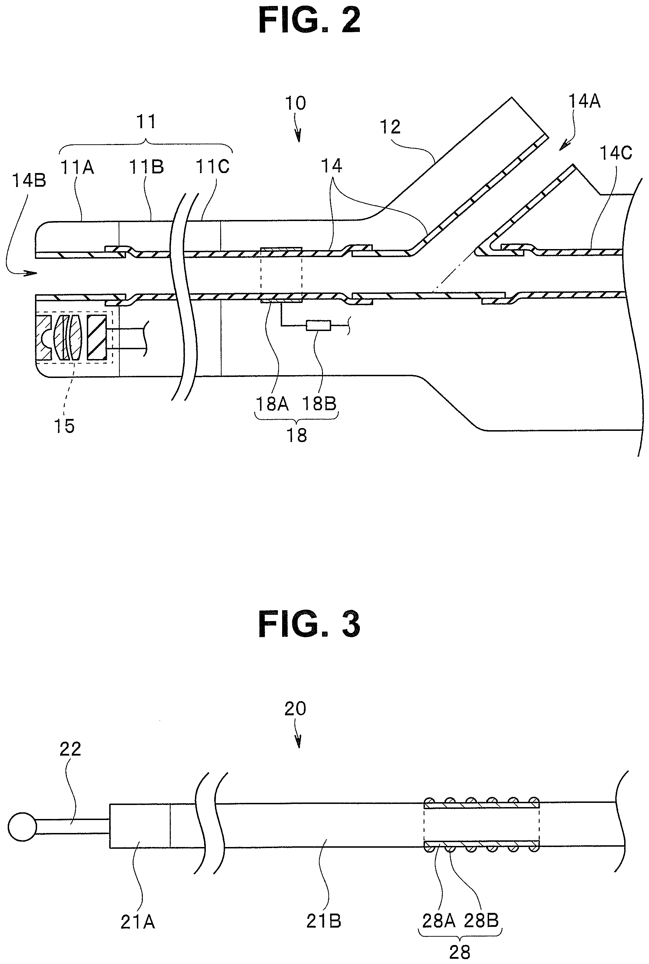

Figs. 1 to 19 relate to a first embodiment of the present invention. Fig. 1 is a diagram showing a configuration of main parts of a medical system according to the first embodiment. Fig. 2 is a cross-sectional schematic diagram of an endoscope of the medical system according to the first embodiment.

As shown for example in Fig. 1, a medical system 1 includes: a flexible endoscope (hereinafter, abbreviated as an endoscope) 10; a treatment device 20 that is a device inserted into a cylindrical channel 14 provided on the endoscope 10; a power source 30; and a counter electrode plate 40.

The endoscope 10 includes: a cylindrical insertion portion 11; an operation portion 12 installed on a proximal end portion side of the insertion portion 11; and a universal cord 13 extended from the operation portion 12.

As shown in Figs. 1 and 2, the insertion portion 11 includes: a distal end portion 11A provided with an image pickup section 15; a bending portion 11B for changing a direction of the distal end portion 11A; and a flexible portion 11C in an elongated shape formed by a flexible member such as a resin.

The operation portion 12 is grasped by a surgeon and is formed as a non-flexible portion that can perform direction operation of the distal end portion 11A, air/water feeding operation, endoscopic image photographing operation, and the like. On the other hand, the insertion portion 11 is formed as a flexible portion inserted into, for example, inside of a digestive tract from an oral cavity or an anus of a patient that is a subject 2.

A processor 32 can be detachably connected to the universal cord 13 of the endoscope 10. The processor 32 includes a control section (not shown) including a CPU or the like that controls the entire medical system 1 and is configured to generate an endoscopic image by processing an image pickup signal outputted by the image pickup section 15 and to output the generated endoscopic image to a monitor 33.

The power source 30 is configured to be able to supply high-frequency power used for treatment by a treatment device 20 (treatment section 22 described later). More specifically, the power source 30 is configured to be able to output high-frequency power with a frequency of, for example, 100 kHz or more and 100 MHz or less. The power source 30 is configured to be able to supply high-frequency power to a power transmission section 18 (described later) of the endoscope 10 through the processor 32, the universal cord 13, and the like. Note that the power source 30 of the present embodiment may be connected to wiring branched from the universal cord 13 to supply high-frequency power without going through the processor 32, for example.

A foot switch 31 is configured to be able to instruct the power source 30 to switch ON/OFF of the output of the high-frequency power from the power source 30 according to operation by the surgeon.

The counter electrode plate 40 is formed by, for example, a stainless metal conductor and can be brought into contact with and attached to a back side of the subject 2 at a wide area. The counter electrode plate 40 is configured to function as an electrode in a return circuit for returning, to the power source 30, high-frequency current applied from the treatment device 20 to living tissue of a site to be treated 2A existing inside the subject 2.

As shown in Figs. 1 and 2, the channel 14 is in connected to an opening 14B of the distal end portion 11A in a state in which a conduit branched toward an insertion port 14A of the operation portion 12 and a conduit branched toward an air suction tube 14C are integrated into one conduit.

And, the endoscope 10 includes the power transmission section 18 that generates an AC electric field according to the high-frequency power supplied from the power source 30, as shown in Fig. 2.

The power transmission section 18 is installed along a cylindrical outer circumferential face of the channel 14 and includes: a power transmission electrode 18A that generates an AC electric field according to the high-frequency power supplied from the power source 30; and an inductor 18B serially connected to the power transmission electrode 18A.

The power transmission electrode 18A is formed by, for example, a cylindrical conductor with a length of about 15 cm and is provided on at least part of a zone from the insertion port 14A to the opening 14B. A surface of the power transmission electrode 18A is covered by an insulator (not shown) such as a resin.

Note that the power transmission section 18 of the present embodiment may include an impedance matching circuit for matching an output impedance of the power source 30 with an input impedance of a circuit including each section connected to the power source 30.

The power transmission electrode 18A of the present embodiment may be, for example, a metal film made of copper formed by applying a deposition method or a plating method to an outer circumferential face of the channel 14 that is a flexible tube.

The treatment device 20 is formed as, for example, a monopolar high-frequency electric cautery. As shown in Figs. 1 and 3, the treatment device 20 includes: a distal end portion 21A provided with the treatment section (cautery electrode) 22; an insertion portion 21B installed on a proximal end side of the distal end portion 21A; and an operation portion 21C installed on a proximal end side of the insertion portion 21B. Fig. 3 is a cross-sectional schematic diagram of the treatment device according to the first embodiment.

The insertion portion 21B is formed by a flexible member such as a resin. The insertion portion 21B is inserted into the channel 14 from the insertion port 14A of the endoscope 10 and is formed in a cylindrical shape that allows insertion into the channel 14.

The operation portion 21C is configured to be able to perform, for example, operation of changing an amount of projection of the treatment section 22 according to operation by the surgeon.

As shown in Fig. 3, the treatment device 20 includes a power reception section 28 including: a power reception electrode 28A installed along a cylindrical outer circumferential face of the insertion portion 21B; and one or a plurality of protruding portions 28B provided at a position including an installation position of the power reception electrode 28A on the outer circumferential face of the insertion portion 21B.

The power reception electrode 28A is formed by, for example, a cylindrical conductor with a length greater than a length of the power transmission electrode 18A. The power reception electrode 28A is connected to the treatment section 22 through a conductor wire (not shown) provided inside the treatment device 20. A surface of the power reception electrode 28A is covered by an insulator (not shown) such as a resin.

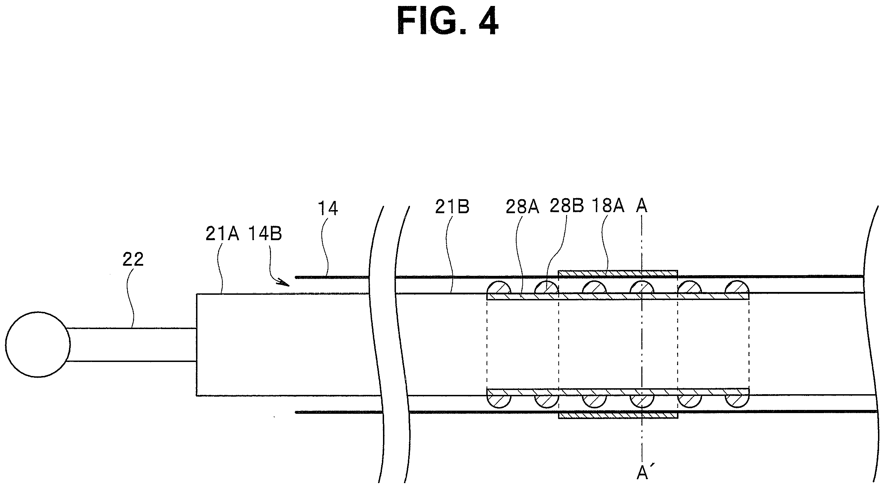

An installation position of the power reception electrode 28A in the insertion portion 21B is positioned in advance to oppose the power transmission electrode 18A when the insertion portion 21B is inserted into the channel 14 and the treatment section 22 protrudes from the opening 14B as shown in Fig. 4, for example. The length of the power reception electrode 28A is about several centimeters greater than the length of the power transmission electrode 18A so as to oppose an entire range of the power transmission electrode 18A in a longitudinal direction even if the treatment device 20 moves forward and backward in the channel 14 during the treatment by the treatment section 22, for example. Fig. 4 is a diagram showing an example of a configuration of protruding portions provided on the treatment device according to the first embodiment.

Note that according to the present embodiment, it is only necessary that insulation is maintained between the two electrodes, the power transmission electrode 18A and the power reception electrode 28A, when the two electrodes are arranged at opposing positions. In other words, according to the present embodiment, it is only necessary that the surface of at least one of the two electrodes, the power transmission electrode 18A and the power reception electrode 28A, is covered by an insulator such as a resin, for example.

The protruding portions 28B are provided to project outside in a radial direction of the insertion portion 21B and have a function as a position restriction section that restricts the position of the insertion portion 21B in the radial direction in the channel 14 so that an amount of eccentricity DQ equivalent to a distance between a center axis of the channel 14 in a longitudinal direction and a center axis of the insertion portion 21B in a longitudinal direction at the installation position of the power reception electrode 28A is equal to or smaller than a predetermined value.

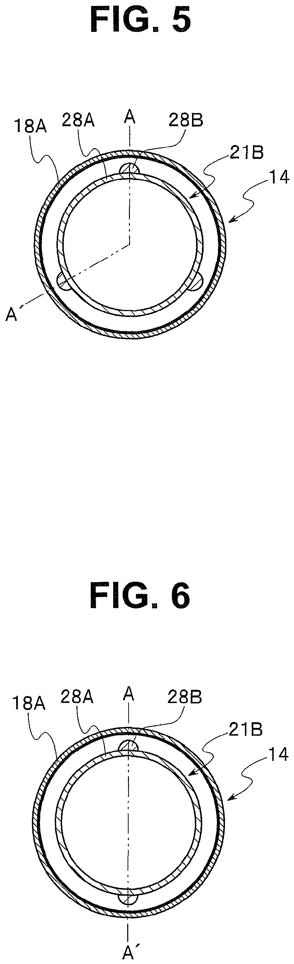

More specifically, as shown for example in Figs. 4 and 5, the protruding portions 28B have a substantially hemispherical shape, and a set of three protruding portions 28B are provided at positions dividing a circumference of the insertion portion 21B into three equal parts. The protruding portions 28B are provided such that intervals of each set are substantially equal intervals in the longitudinal direction of the insertion portion 21B. Alternatively, as shown for example in Figs. 4 and 6, the protruding portions 28B have a substantially hemispherical shape, and a set of two protruding portions 28B are provided at positions dividing the circumference of the insertion portion 21B into two equal parts. The protruding portions 28B are provided such that the intervals of each set are substantially equal intervals in the longitudinal direction of the insertion portion 21B. Fig. 5 is a cross-sectional view showing an example of a cross section cut along a line A-A'. Fig. 6 is a cross-sectional view showing an example of the cross section cut along the line A-A', the example different from Fig. 5.

According to the present embodiment, the protruding portions 28B can be, for example, in point contact with an inner circumferential face of the channel 14, and slidability of the treatment device 20 (insertion portion 21B) in the channel 14 can be secured.

Subsequently, action of the present embodiment will be described.

In a state that the distal end portion 11A of the endoscope 10 is arranged near the site to be treated 2A existing inside the subject 2, the surgeon inserts the treatment device 20 into the channel 14 to cause the treatment section 22 to project from the opening 14B. Then, in a state that the treatment section 22 is in contact with the living tissue of the site to be treated 2A, the surgeon operates the foot switch 31 to perform an instruction for turning on the output of the high-frequency power from the power source 30. The high-frequency power is supplied from the power source 30 to the power transmission section 18 according to the instruction.

Here, when the insertion portion 21B is inserted into the channel 14 and the treatment section 22 projects from the opening 14B as illustrated in Fig. 4, a capacitor C1 is formed by capacitive coupling of the two electrodes, the power transmission electrode 18A and the power reception electrode 28A, caused by the opposing arrangement of the two electrodes, and power from the AC electric field generated in the power transmission electrode 18A is fed through the capacitor C1. Therefore, the high-frequency power can be wirelessly supplied from the power transmission section 18 to the power reception section 28 along with the formation of the capacitor.

When the insertion portion 21B is inserted into the channel 14 and the treatment section 22 projects from the opening 14B, an LC resonant circuit is formed by the capacitor C1 and the inductor 18B serially connected to the power transmission electrode 18A. Therefore, for example, each section of the power transmission section 18 and the power reception section 28 can be formed such that a resonant frequency in the LC resonant circuit is a predetermined frequency such as 13.56 MHz, and a frequency of the high-frequency power supplied from the power source 30 to the power transmission section 18 can be brought into line with or substantially brought into line with the predetermined frequency. In this way, the power can be efficiently supplied from the power transmission section 18 to the power reception section 28.

The high-frequency power supplied from the power transmission section 18 is received by the power reception section 28 and then supplied to the treatment section 22 through the conductor wire (not shown) provided inside the treatment device 20. Along with the application of the high-frequency current from the treatment section 22 to the living tissue of the site to be treated 2A, the treatment section 22 and the counter electrode plate 40 (return circuit including the counter electrode plate 40) are energized, and the site to be treated 2A is treated by Joule heat generated according to the energization.

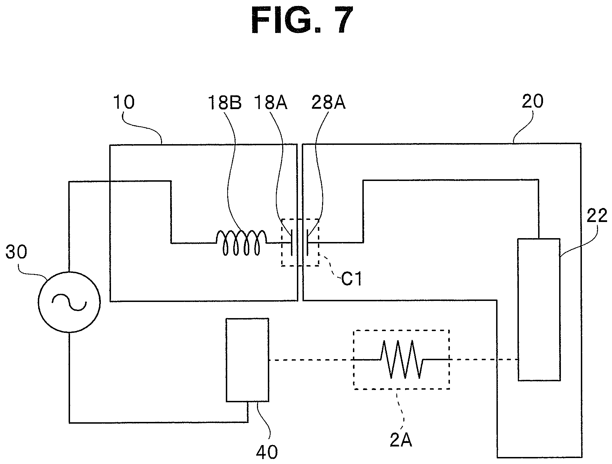

Note that the living tissue of the site to be treated 2A functions as a resistance in an electric circuit. Therefore, each section regarding the treatment of the living tissue of the site to be treated 2A can be illustrated as an equivalent circuit as shown in Fig. 7. Fig. 7 is a diagram showing the equivalent circuit of main parts of the medical system according to the first embodiment.

Even if the surgeon moves forward and backward or rotates the treatment device 20 (insertion portion 21B) in the channel 14 during the treatment by the treatment section 22 for example, the position of the insertion portion 21B in the channel 14 in the radial direction is restricted (such that the amount of eccentricity DQ is equal to or smaller than the predetermined value), as the protruding portions 28B come into contact with the inner circumferential face of the channel 14.

As described, according to the present embodiment, the capacitive coupling of the power transmission electrode 18A and the power reception electrode 28A can be utilized to wirelessly supply the power from the endoscope 10 to the treatment device 20 without connecting a cable for supplying power to the treatment device 20.

According to the present embodiment, even if the surgeon moves forward and backward or rotates the treatment device 20 (insertion portion 21B) in the channel 14 during the treatment by the treatment section 22, the position of the insertion portion 21B in the channel 14 in the radial direction is restricted such that the amount of eccentricity DQ is equal to or smaller than the predetermined value. Therefore, fluctuation of electrostatic capacity of the capacitor C1 formed by the capacitive coupling of the power transmission electrode 18A and the power reception electrode 28A can be prevented as much as possible. Specifically, for example, an inner diameter of the channel 14 is 2.8 mm, an outer diameter of the insertion portion 21B is 2.5 mm, a material of the insulator covering the surfaces of the power transmission electrode 18A and the power reception electrode 28A is a fluoro resin, and a thickness of the fluoro resin is 0.05 mm. In this case, the protruding portions 28B can be formed such that the amount of eccentricity DQ is 75 µm or less, and the fluctuation of the electrostatic capacity of the capacitor C1 can be prevented to a level of up to about 8%. Furthermore, for example, the inner diameter of the channel 14 is 2.8 mm, the outer diameter of the insertion portion 21B is 2.5 mm, the material of the insulator covering the surfaces of the power transmission electrode 18A and the power reception electrode 28A is a fluoro resin, and the thickness of the fluoro resin is 0.05 mm. In this case, the protruding portions 28B can be formed such that the amount of eccentricity DQ is 50 µm or less, and the fluctuation of the electrostatic capacity of the capacitor C1 can be prevented to a level of up to about 4%. That is, according to the present embodiment, the fluctuation of the impedance of the circuit including a power transmission section 19 and a power reception section 29 as viewed from the power source 30 can be prevented, and the fluctuation of transmission power to the treatment device 20 associated with the fluctuation of the impedance can be prevented. Furthermore, according to the present embodiment, the protruding portions 28B come in point contact with the inner circumferential face of the channel 14, and the slidability of the treatment device 20 (insertion portion 21B) in the channel 14 can be secured. This can prevent reduction in operability when the treatment device 20 (insertion portion 21B) is operated by inserting the treatment device 20 into the channel 14.

Therefore, the present embodiment can stabilize a supply state of power in the wireless power feeding, while preventing the reduction in the operability when the treatment device is used by inserting the treatment device into the channel of the endoscope.

Note that according to the present embodiment, substantially the same effects can also be attained when there is no inductor 18B serially connected to the power transmission electrode 18A, that is, when the LC resonant circuit including the capacitor C1 is not formed.

By the way, according to the present embodiment, the shape, the installed position, and/or the number of installed protruding portions 28B may be appropriately changed as long as the configuration satisfies the function of the position restriction section.

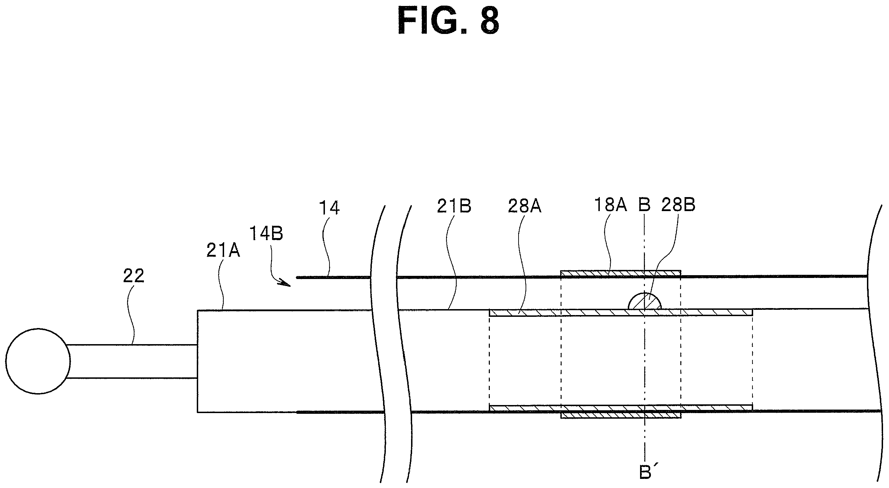

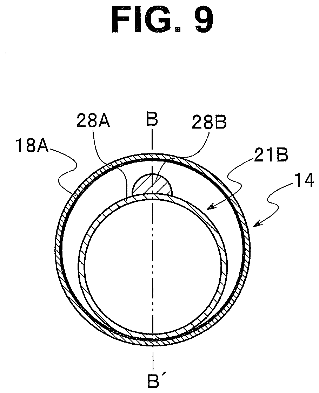

More specifically, according to the present embodiment, the protruding portion 28B may be provided only at one part on the outer circumferential face of the insertion portion 21B as shown for example in Figs. 8 and 9. Fig. 8 is a diagram showing an example of the configuration of the protruding portion provided on the treatment device according to the first embodiment, the example different from Fig. 4. Fig. 9 is a cross-sectional view showing a cross section cut along a line B-B'.

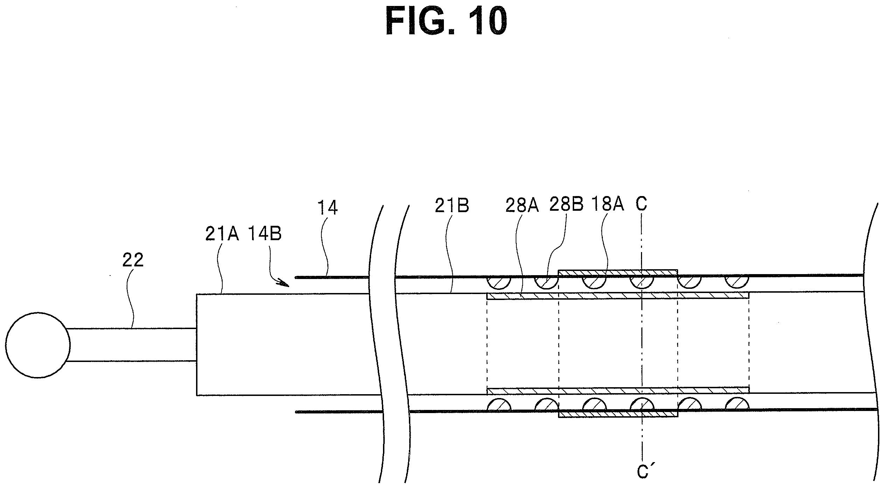

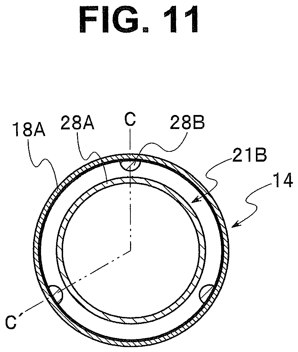

Alternatively, according to the present embodiment, the protruding portions 28B may be provided on the inner circumferential face of the channel 14 as shown for example in Figs. 10 and 11, instead of providing the protruding portions 28B on the outer circumferential face of the insertion portion 21B. Fig. 10 is a diagram showing an example of the configuration of the protruding portions provided on the treatment device according to the first embodiment, the example different from Figs. 4 and 8. Fig. 11 is a cross-sectional view showing a cross section cut along a line C-C'.

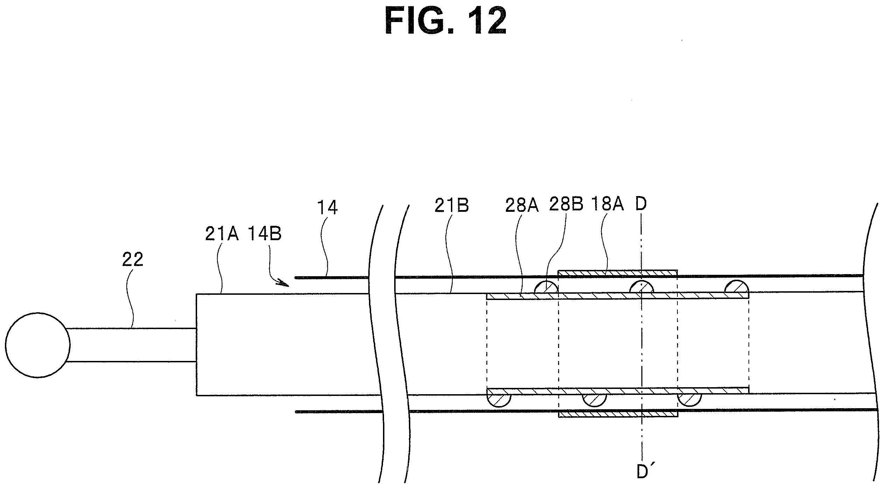

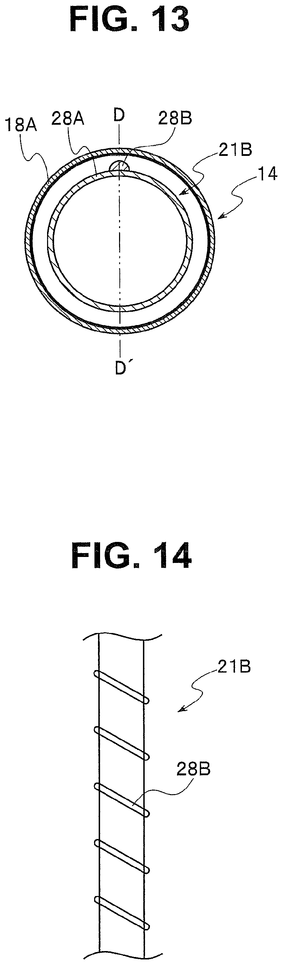

Alternatively, according to the present embodiment, the protruding portions 28B may be alternately provided in the longitudinal direction of the insertion portion 21B as shown for example in Figs. 12 and 13, at two positions dividing the circumference of the insertion portion 21B into two equal parts. Fig. 12 is a diagram showing an example of the configuration of the protruding portions provided on the treatment device according to the first embodiment, the example different from Figs. 4, 8, and 10. Fig. 13 is a cross-sectional view of a cross section cut along a line D-D'.

Alternatively, according to the present embodiment, a protruding portion 28C formed in a spiral shape may be provided in the longitudinal direction of the insertion portion 21B as shown for example in Fig. 14, in place of the protruding portions 28B formed in a substantially hemispherical shape. Fig. 14 is a diagram showing an example of the configuration of the protruding portion provided on the treatment device according to the first embodiment, the example different from Figs. 4, 8, 10, and 12.

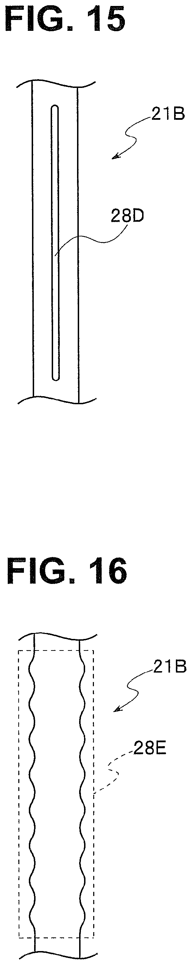

Alternatively, according to the present embodiment, a protruding portion 28D formed in a linear shape may be provided in the longitudinal direction of the insertion portion 21B as shown for example in Fig. 15, in place of the protruding portions 28B formed in a substantially hemispherical shape. Fig. 15 is a diagram showing an example of the configuration of the protruding portion provided on the treatment device according to the first embodiment, the example different from Figs. 4, 8, 10, 12, and 14.

Alternatively, according to the present embodiment, a protruding portion 28E formed in a waveform shape may be provided in the longitudinal direction of the insertion portion 21B as shown for example in Fig. 16, in place of the protruding portions 28B formed in a substantially hemispherical shape. Fig. 16 s a diagram showing an example of the configuration of the protruding portion provided on the treatment device according to the fist embodiment, the example different from Figs. 4, 8, 10, 12, 14, and 15.

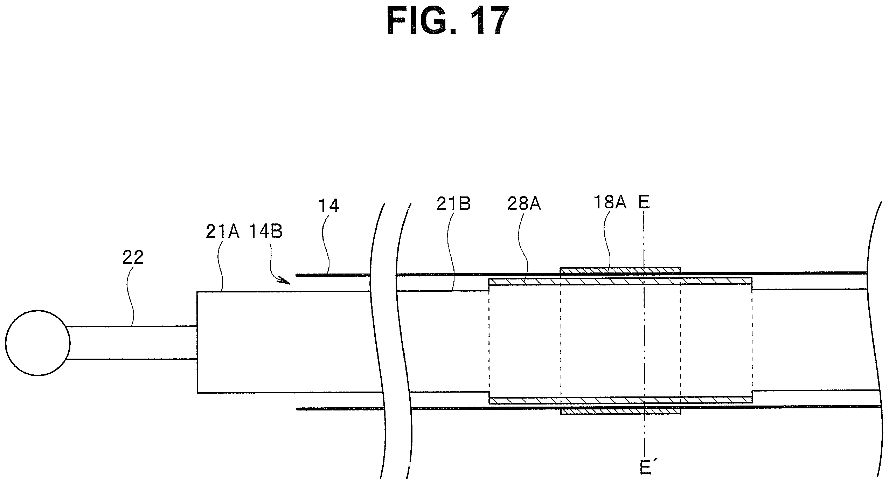

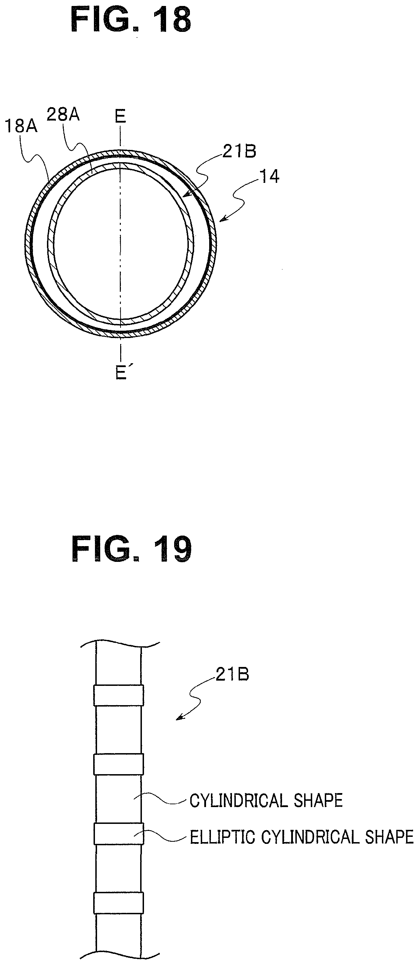

On the other hand, according to the present embodiment, the position restriction section may be formed by applying processing of deforming a part of the insertion portion 21B equivalent to the installation position of the power reception electrode 28A from a cylindrical shape to an elliptic cylindrical shape as shown for example in Figs. 17 and 18, instead of providing the protruding portions 28B on the outer circumferential face of the insertion portion 21B. Note that when the processing is applied, a major axis direction of the elliptic cylinder can satisfy the function of the position restriction section. Fig. 17 is a schematic diagram showing an example in which part of the insertion portion of the treatment device in the medical system according to the first embodiment is deformed into an elliptic cylindrical shape. Fig. 18 is a cross-sectional view showing a cross section cut along a line E-E'.

Furthermore, the processing may be applied to a part or a plurality of parts of the portion equivalent to the installation position of the power reception electrode 28A in the insertion portion 21B to deform the plurality of parts into an elliptic cylindrical shape as shown for example in Fig. 19. Fig. 19 is a schematic diagram showing an example different from Fig. 17, in which part of the insertion portion of the treatment device in the medical system according to the first embodiment is deformed into an elliptic cylindrical shape.

Alternatively, according to the present embodiment, the position restriction section may be formed by, for example, applying processing of deforming the part equivalent to the installation position of the power transmission electrode 18A in the channel 14 from a cylindrical shape to an elliptic cylindrical shape, instead of providing the protruding portions 28B on the outer circumferential face of the insertion portion 21B. Note that when the processing is applied, a short axis direction of the elliptic cylinder can satisfy the function of the position restriction section.

### (Second Embodiment)

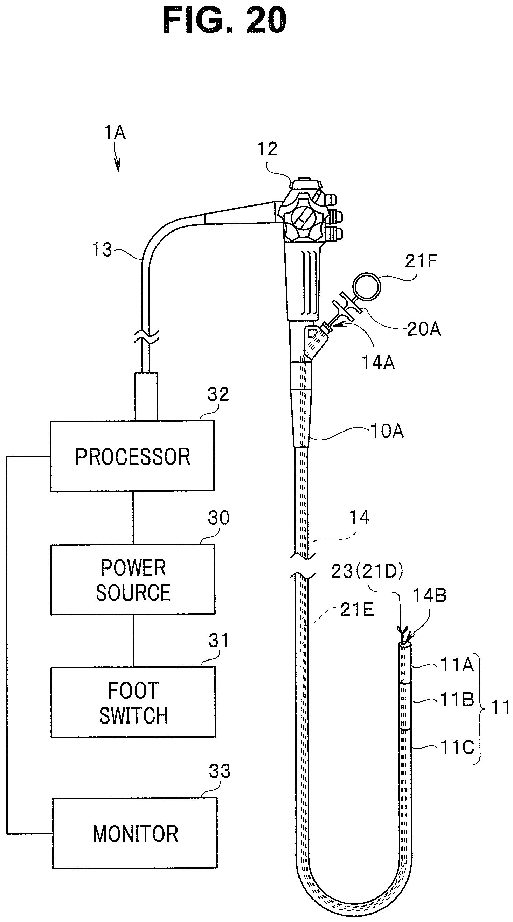

Figs. 20 to 26 relate to a second embodiment of the present invention.

Note that details related to the part with the same components and the like as in the first embodiment will not be described in the present embodiment, and part with components and the like different from the first embodiment will be mainly described.

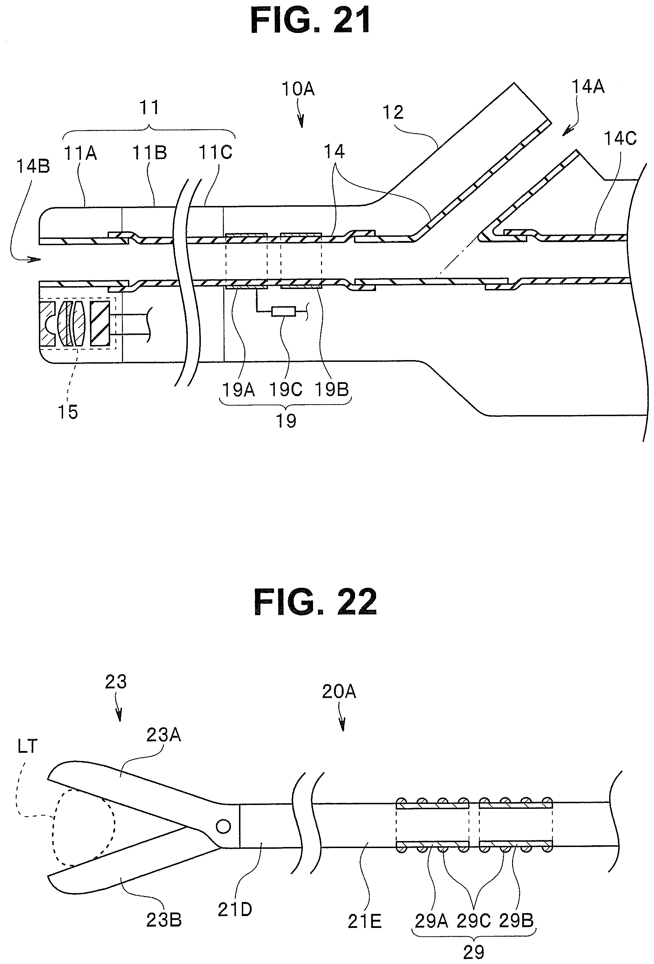

Fig. 20 is a diagram showing a configuration of main parts of a medical system according to the second embodiment. Fig. 21 is a cross-sectional schematic diagram of an endoscope of the medical system according to the second embodiment.

As shown for example in Fig. 20, a medical system 1A includes: a flexible endoscope (hereinafter, abbreviated as an endoscope) 10A; a treatment device 20A that is a device inserted into the channel 14 of the endoscope 10A; and the power source 30.

As shown in Fig. 21, the endoscope 10A is provided with the power transmission section 19 that generates an AC electric field according to the high-frequency power supplied from the power source 30, in place of the power transmission section 18 in the endoscope 10.

The power transmission section 19 is installed along the outer circumferential face of the channel 14 and includes: a first power transmission electrode 19A and a second power transmission electrode 19B, each generating an AC electric field according to the high-frequency power supplied from the power source 30; and an inductor 19C serially connected to the first power transmission electrode 19A.

Each of the first power transmission electrode 19A and the second power transmission electrode 19B is formed by, for example, a cylindrical conductor and is provided in the zone from the insertion port 14A to the opening 14B. Each of the surfaces of the first power transmission electrode 19A and the second power transmission electrode 19B is covered by an insulator (not shown) such as a resin.

The first power transmission electrode 19A and the second power transmission electrode 19B of the present embodiment may be, for example, metal films made of copper formed by applying a deposition method or a plating method to the outer circumferential face of the channel 14 that is a flexible tube.

The treatment device 20A is formed as, for example, a bipolar high-frequency electric cautery. As shown in Figs. 20 and 22, the treatment device 20A includes: a distal end portion 21D provided with a treatment section 23 as forceps; an insertion portion 21E installed on a proximal end side of the distal end portion 21D; and an operation portion 21F installed on a proximal end side of the insertion portion 21E. Fig. 22 is a cross-sectional schematic diagram of the treatment device according to the second embodiment.

The insertion portion 21E is formed by a flexible member such as a resin. The insertion portion 21E is inserted into the channel 14 from the insertion port 14A of the endoscope 10A and is formed in a cylindrical shape that allows insertion to the channel 14.

The operation portion 21F is configured to be able to perform, for example, operation of opening and closing a pair of blades 23A and 23B of the treatment section 23 according to operation by the surgeon. That is, the pair of blades 23A and 23B of the treatment device 20A can sandwich a living tissue LT of the site to be treated.

As shown in Fig. 22, the treatment device 20A includes the power reception section 29 including; a first power reception electrode 29A and a second power reception electrode 29B installed along an outer circumferential face of the insertion portion 21E; and one or a plurality of protruding portions 29C provided at positions including installation positions of the first power reception electrode 29A and the second power reception electrode 29B on the outer circumferential face of the insertion portion 21E.

Each of the first power reception electrode 29A and the second power reception electrode 29B is formed by, for example, a cylindrical conductor with a length greater than the length of the first power transmission electrode 19A and the second power transmission electrode 19B. The first power reception electrode 29A is connected to the blade 23A through a conductor wire (not shown) provided inside the treatment device 20A. The second power reception electrode 29B is connected to the blade 23B through a conductor wire (not shown) provided inside the treatment device 20A. Each of the surfaces of the first power reception electrode 29A and the second power reception electrode 29B is covered by an insulator (not shown) such as a resin.

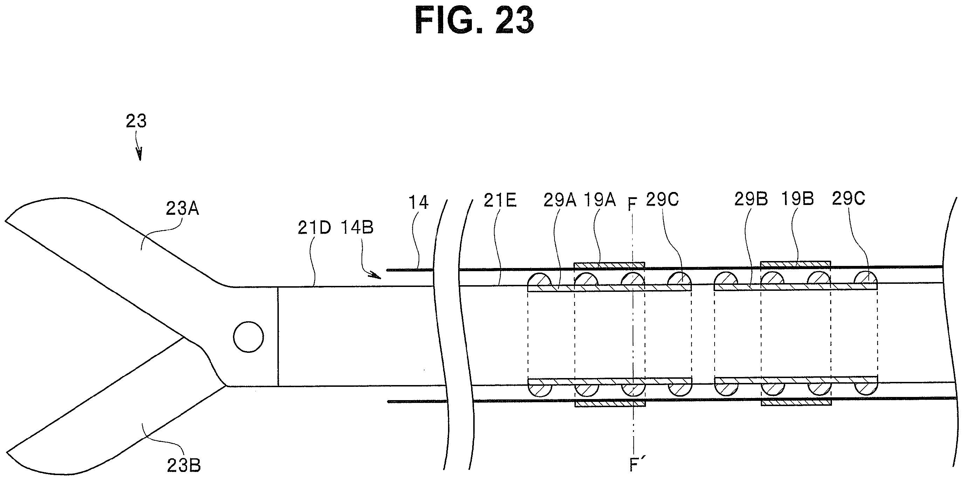

The installation position of the first power reception electrode 29A in the insertion portion 21E is positioned in advance to oppose the first power transmission electrode 19A when the insertion portion 21E is inserted into the channel 14 until the treatment section 23 projects from the opening 14B as shown in Fig. 23, for example. The length of the first power reception electrode 29A is about several centimeters greater than the length of the first power transmission electrode 19A so as to oppose an entire range of the first power transmission electrode 19A in a longitudinal direction even if the treatment device 20A moves forward and backward in the channel 14 during the treatment by the treatment section 23, for example. Fig. 23 is a diagram showing an example of a configuration of protruding portions provided on the treatment device according to the second embodiment.

Note that according to the present embodiment, it is only necessary that insulation is maintained between the two electrodes, the first power transmission electrode 19A and the first power reception electrode 29A, when the two electrodes are arranged at opposing positions. In other words, according to the present embodiment, it is only necessary that the surface of at least one of the two electrodes, the first power transmission electrode 19A and the first power reception electrode 29A, is covered by an insulator such as a resin, for example.

The installation position of the second power reception electrode 29B in the insertion portion 21E is positioned in advance to oppose the second power transmission electrode 19B when the insertion portion 21E is inserted into the channel 14 until the treatment section 23 projects from the opening 14B as shown in Fig. 23, for example. The length of the second power reception electrode 29B is about several centimeters greater than the length of the second power transmission electrode 19B so as to oppose an entire range of the second power transmission electrode 19B in a longitudinal direction even if the treatment device 20A moves forward and backward in the channel 14 during the treatment by the treatment section 23, for example.

Note that according to the present embodiment, it is only necessary that insulation is maintained between the two electrodes, the second power transmission electrode 19B and the second power reception electrode 29B, when the two electrodes are arranged at opposing positions. In other words, according to the present embodiment, it is only necessary that the surface of at least one of the two electrodes, the second power transmission electrode 19B and the second power reception electrode 29B, is covered by an insulator such as a resin, for example.

The protruding portions 29C are provided to project outside in a radial direction of the insertion portion 21E and have a function as a position restriction section that restricts the position of the insertion portion 21E in the radial direction in the channel 14 so that each of an amount of eccentricity DQA equivalent to a distance between the center axis of the channel 14 in the longitudinal direction and a center axis of the insertion portion 21E in a longitudinal direction at the installation position of the first power reception electrode 29A and an amount of eccentricity DQB equivalent to a distance between the center axis of the channel 14 in the longitudinal direction and the center axis of the insertion portion 21E in the longitudinal direction at the installation position of the second power reception electrode 29B is equal to or smaller than a predetermined value.

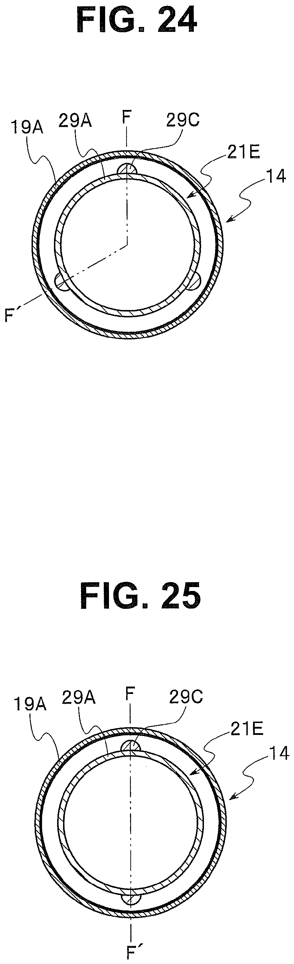

More specifically, as shown for example in Figs. 23 and 24, the protruding portions 29C have a substantially hemispherical shape, and a set of three protruding portions 29C are provided at positions dividing a circumference of the insertion portion 21E into three equal parts. The protruding portions 29C are provided such that intervals of each set are substantially equal intervals in the longitudinal direction of the insertion portion 21E. Alternatively, as shown for example in Figs. 23 and 25, the protruding portions 29C have a substantially hemispherical shape, and a set of two protruding portions 29C are provided at positions dividing the circumference of the insertion portion 21E into two equal parts. The protruding portions 29C are provided such that the intervals of each set are substantially equal intervals in the longitudinal direction of the insertion portion 21E.

Note that according to the present embodiment, the protruding portions 28E can be, for example, in point contact with an inner circumferential face of the channel 14, and the slidability of the treatment device 20A (insertion portion 21E) in the channel 14 can be secured.

Subsequently, action of the present embodiment will be described.

In a state that the distal end portion 11A of the endoscope 10A is arranged near the site to be treated inside the subject 2, the surgeon inserts the treatment device 20A into the channel 14 to cause the treatment section 23 to project from the opening 14B. Then, in a state that the blades 23A and 23B sandwich the living tissue LT of the site to be treated, the surgeon operates the foot switch 31 to perform an instruction for turning on the output of the high-frequency power from the power source 30. The high-frequency power is supplied from the power source 30 to the power transmission section 19 according to the instruction.

Here, when the insertion portion 21E is inserted into the channel 14 until the treatment section 23 projects from the opening 14B as illustrated in Fig. 23, a capacitor C2 is formed by capacitive coupling of the two electrodes, the first power transmission electrode 19A and the first power reception electrode 29A, caused by the opposing arrangement of the two electrodes. A capacitor C3 is also formed by capacitive coupling of the two electrodes, the second power transmission electrode 19B and the second power reception electrode 29B, caused by the opposing arrangement of the two electrodes. The power reception section 29 of the treatment device 20A is capacitively coupled to the power transmission section 19 of the endoscope 10A along with the formation of the capacitors C2 and C3. As a result, the high-frequency power outputted from the power source 30 can be wirelessly supplied from the power transmission section 19 to the power reception section 29 through the capacitors C2 and C3.

When the insertion portion 21E is inserted into the channel 14 until the treatment section 23 projects from the opening 14B, an LC resonant circuit is formed by the capacitors C2 and C3 and the inductor 19C serially connected to the first power transmission electrode 19A. Therefore, for example, each section of the power transmission section 19 and the power reception section 29 can be formed such that a resonant frequency in the LC resonant circuit is a predetermined frequency such as 13.56 MHz, and a frequency of the high-frequency power supplied from the power source 30 to the power transmission section 19 can be brought into line with or substantially brought into line with the predetermined frequency. In this way, the power can be efficiently supplied from the power transmission section 19 to the power reception section 29.

The high-frequency power supplied from the power transmission section 19 is received by the power reception section 29 and then supplied to the treatment section 23 through the conductor wire (not shown) provided inside the treatment device 20A. Along with the application of the high-frequency current from the treatment section 23 to the living tissue LT, the blade 23A and the blade 23B are energized, and the living tissue LT is treated by Joule heat generated according to the energization.

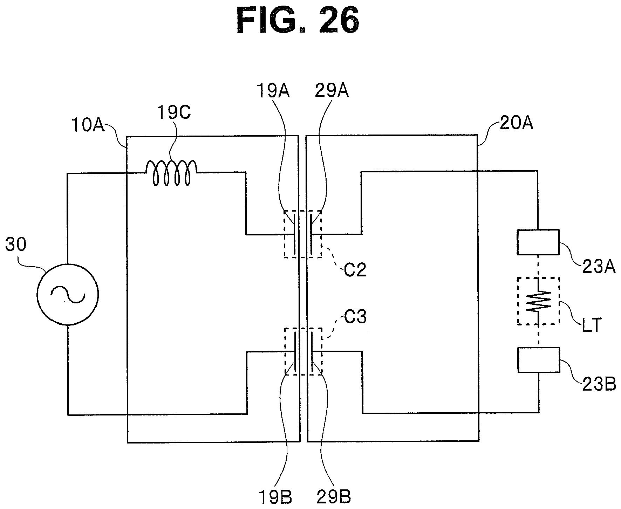

Note that the living tissue LT in the site to be treated functions as a resistance in an electric circuit. Therefore, each section regarding the treatment of the living tissue LT can be illustrated as an equivalent circuit as shown in Fig. 26. Fig. 26 is a diagram showing the equivalent circuit of main parts of the medical system according to the second embodiment.

And, even if the surgeon moves forward and backward or rotates the treatment device 20A (insertion portion 21E) in the channel 14 during the treatment by the treatment section 23 for example, the position of the insertion portion 21E in the channel 14 in the radial direction is restricted (such that each of the amounts of eccentricity DQA and DQB is equal to or smaller than the predetermined value), as the protruding portions 29C come into contact with the inner circumferential face of the channel 14.

As described, according to the present embodiment, the capacitive coupling of the first power transmission electrode 19A and the first power reception electrode 29A as well as the capacitive coupling of the second power transmission electrode 19B and the second power reception electrode 29B can be utilized to wirelessly supply the power from the endoscope 10A to the treatment device 20A without connecting a cable for supplying power to the treatment device 20A.

According to the present embodiment, even if the surgeon moves forward and backward or rotates the treatment device 20A (insertion portion 21E) in the channel 14 during the treatment by the treatment section 23, the position of the insertion portion 21E in the channel 14 in the radial direction is restricted such that the amount of eccentricity DQA and the amount of eccentricity DQB are equal to or smaller than the predetermined value. Therefore, fluctuation of electrostatic capacity of the capacitor C2 formed by the capacitive coupling of the first power transmission electrode 19A and the first power reception electrode 29A as well as the capacitor C3 formed by the capacitive coupling of the second power transmission electrode 19B and the second power reception electrode 29B can be prevented as much as possible. That is, according to the present embodiment, the fluctuation of the impedance of the circuit including the power transmission section 19 and the power reception section 29 as viewed from the power source 30 can be prevented, and the fluctuation of transmission power to the treatment device 20A associated with the fluctuation of the impedance can be prevented. Furthermore, according to the present embodiment, the protruding portions 29C come in point contact with the inner circumferential face of the channel 14, and the slidability of the treatment device 20A (insertion portion 21E) in the channel 14 can be secured. This can prevent reduction in operability when the treatment device 20A (insertion portion 21E) is operated by inserting the treatment device 20A into the channel 14.

Therefore, the present embodiment can stabilize a supply state of power in the wireless power feeding, while preventing the reduction in the operability when the treatment device is used by inserting the treatment device into the channel of the endoscope.

Note that according to the present embodiment, substantially the same effects can also be attained when there is no inductor 19C serially connected to the first power transmission electrode 19A, that is, when the LC resonant circuit including the capacitors C2 and C3 is not formed.

The present invention is not limited to each of the embodiments, and it is obvious that various changes and applications can be made without departing from the scope of the invention.

## Claims

1. A medical system (1) comprising:
an endoscope (10) including a cylindrical channel (14);
a treatment device (20) configured to be inserted into the channel (14) from an insertion port (14A) of the endoscope (10); and
a power source (30) configured to supply high-frequency power used for treatment by the treatment device (20), wherein
the endoscope (10) comprises a power transmission electrode (18) installed along an outer circumferential face of the channel (14) and configured to generate an AC electric field according to the high-frequency power supplied from the power source (30), and
the treatment device (20) comprises:
a cylindrical insertion portion (21B) insertable into the channel (14);
a treatment section (22) installed on a distal end portion (21A) of the insertion portion (21B) and configured to perform treatment according to high-frequency power supplied from the power source (30) in a state that the insertion portion (21B) is inserted into the channel (14) so that the distal end portion is projected from an opening (14B) different from the insertion port (14A);
a power reception electrode (28) installed along an outer circumferential face of the insertion portion (21B) and configured to form a capacitor (C1) by capacitively coupling to the power transmission electrode (18) when the insertion portion (21B) is inserted into the channel (14) and the treatment section (22) projects from the opening (14B); and
one or a plurality of protruding portions (28B) provided at a position including the installation position of the power reception electrode (28A), wherein the one or the plurality of protruding portions (28B) is configured to restrict a position of the insertion portion (21B) in the channel (14) in a radial direction such that an amount of eccentricity equivalent to a distance between a center axis of the channel (14) in a longitudinal direction and a center axis of the insertion portion (21B) in a longitudinal direction at an installation position of the power reception electrode (28) is equal to or smaller than a predetermined value,
wherein the one or the plurality of protruding portions (28B) is configured to be in point contact with an inner circumferential face of the channel (14).

2. The medical system (1) according to claim 1, wherein
the one or the plurality of protruding portions (28B) projecting outwardly in the radial direction of the insertion portion (21B).

3. The medical system (1) according to claim 2, wherein
the one or the plurality of protruding portions (28B) have one of a substantially hemispherical shape, a spiral shape, a linear shape, and a waveform shape.

4. The medical system (1) according to claim 1, wherein
the position restriction section (28B) includes an elliptic cylindrical shape, in at least part of a zone equivalent to the installation position of the power reception electrode (28) in the insertion portion (21B).

5. The medical system (1) according to claim 1, further comprising:
an inductor (18B) serially connected to the power transmission electrode (18).

## Patentansprüche

1. Medizinisches System (1), umfassend:
Endoskop (10), umfassend einen zylindrischen Kanal (14);
eine Behandlungsvorrichtung (20), die dazu ausgelegt ist, von einer Einsetzöffnung (14A) des Endoskops (10) in den Kanal (14) eingesetzt zu werden; und
eine Energiequelle (30), die dazu ausgelegt ist, Hochfrequenzenergie, die zur Behandlung von der Behandlungsvorrichtung (20) genutzt wird, zuzuführen, wobei
das Endoskop (10) eine Energieübertragungselektrode (18) umfasst, die entlang einer Außenumfangsfläche des Kanals (14) installiert und dazu ausgelegt ist, ein elektrisches Wechselfeld entsprechend der von der Energiequelle (30) zugeführten Hochfrequenzenergie zu erzeugen, und
die Behandlungsvorrichtung (20) umfasst:
einen zylindrischen Einsetzabschnitt (21B), der in den Kanal (14) eingesetzt werden kann;
einen Behandlungsabschnitt (22), der an einem Distalendabschnitt (21A) des Einsetzabschnitts (21B) installiert und dazu ausgelegt ist, eine Behandlung entsprechend der von der Energiequelle (30) zugeführten Hochfrequenzenergie in einem Zustand durchzuführen, in dem der Einsetzabschnitt (21B) derart in den Kanal (14) eingesetzt ist, dass der Distalendabschnitt von einer Öffnung (14B), die sich von der Einsetzöffnung (14A) unterscheidet, vorspringt;
eine Energieempfangselektrode (28), die entlang einer Außenumfangsfläche des Einsetzabschnitts (21B) installiert und dazu ausgelegt ist, durch kapazitives Koppeln mit der Energieübertragungselektrode (18) einen Kondensator (C1) zu bilden, wenn der Einsetzabschnitt in den Kanal (14) eingesetzt wird und der Behandlungsabschnitt (22) von der Öffnung (14B) vorspringt; und
einen oder eine Vielzahl von vorspringenden Abschnitten (28B), die in einer Position vorgesehen sind, die die Installationsposition der Energieempfangselektrode (28A) umfasst, wobei der eine oder die Vielzahl von vorspringenden Abschnitten (28B) dazu ausgelegt ist, eine Position des Einsetzabschnitts (21B) in dem Kanal (14) derart in einer radialen Richtung zu begrenzen, dass ein Exzentrizitätsbetrag, der zu einem Abstand zwischen einer Mittelachse des Kanals (14) in einer Längsrichtung und einer Mittelachse des Einsetzabschnitts (21B) in einer Längsrichtung in einer Installationsposition der Energieempfangselektrode (28), gleich oder geringer ist als ein vorbestimmter Wert,
wobei der eine oder die Vielzahl von vorspringenden Abschnitten (28B) dazu ausgelegt ist, in Punktkontakt mit einer Innenumfangsfläche des Kanals (14) zu sein.

2. Medizinisches System (1) nach Anspruch 1, wobei
der eine oder die Vielzahl von vorspringenden Abschnitten (28B) nach außen in die radiale Richtung des Einsetzabschnitts (21B) vorspringt.

3. Medizinisches System (1) nach Anspruch 2, wobei
der eine oder die Vielzahl von vorspringenden Abschnitten (28B) eine von einer im Wesentlichen Halbkugelform, einer Spiralform, einer Linearform und einer Wellenform aufweist.

4. Medizinisches System (1) nach Anspruch 1, wobei
der Positionsbegrenzungsabschnitt (28B) eine elliptische zylindrische Form umfasst, in mindestens einem Teil einer Zone, die zu der Installationsposition der Energieempfangselektrode (28) in dem Einsetzabschnitt (21B) äquivalent ist.

5. Medizinisches System (1) nach Anspruch 1, ferner umfassend: einen Induktor (18B), der mit der Energieübertragungselektrode (18) in Reihe geschaltet ist.

## Revendications

1. Système médical (1) comprenant :
un endoscope (10) comprenant un canal cylindrique (14) ;
un dispositif de traitement (20) configuré pour être inséré dans le canal (14) à partir d'un orifice d'insertion (14A) de l'endoscope (10) ; et
une source d'alimentation (30) configurée pour fournir de l'énergie à haute fréquence utilisée pour un traitement par le dispositif de traitement (20),
l'endoscope (10) comprenant une électrode de transmission d'énergie (18) installée le long d'une face circonférentielle externe du canal (14) et configurée pour générer un champ électrique CA selon l'énergie à haute fréquence fournie par la source d'alimentation (30), et le dispositif de traitement (20) comprenant :
une partie d'insertion cylindrique (21B) apte à être insérée dans le canal (14) ;
une section de traitement (22) installée sur une partie d'extrémité distale (21A) de la partie d'insertion (21B) et configurée pour réaliser un traitement selon l'énergie à haute fréquence fournie par la source d'alimentation (30) dans un état dans lequel la partie d'insertion (21B) est insérée dans le canal (14) de telle sorte que la partie d'extrémité distale fait saillie à partir d'une ouverture (14B) différente de l'orifice d'insertion (14A) ;
une électrode de réception d'énergie (28) installée le long d'une face circonférentielle externe de la partie d'insertion (21B) et configurée pour former un condensateur (C1) par couplage capacitif à l'électrode de transmission d'énergie (18) lorsque la partie d'insertion (21B) est insérée dans le canal (14) et la section de traitement (22) fait saillie à partir de l'ouverture (14B) ; et
une ou une pluralité de parties en saillie (28B) prévues dans une position comprenant la position d'installation de l'électrode de réception d'énergie (28A), la ou la pluralité de parties en saillie (28B) étant configurées pour limiter une position de la partie d'insertion (21B) dans le canal (14) dans une direction radiale de telle sorte qu'une quantité d'excentricité équivalente à une distance entre un axe central du canal (14) dans une direction longitudinale et un axe central de la partie d'insertion (21B) dans une direction longitudinale dans une position d'installation de l'électrode de réception d'énergie (28) est inférieure ou égale à une valeur prédéterminée, la ou la pluralité de parties en saillie (28B) étant configurée pour être en contact ponctuel avec une face circonférentielle interne du canal (14).

2. Système médical (1) selon la revendication 1, dans lequel
la ou la pluralité de parties en saillie (28B) font saillie vers l'extérieur dans la direction radiale de la partie d'insertion (21B).

3. Système médical (1) selon la revendication 2, dans lequel
la ou la pluralité de parties en saillie (28B) ont une parmi forme sensiblement hémisphérique, une forme en spirale, une forme linéaire et une forme d'onde.

4. Système médical (1) selon la revendication 1, dans lequel
la section de limitation de position (28B) comprend une forme cylindrique elliptique, dans au moins une partie d'une zone équivalente à la position d'installation de l'électrode de réception d'énergie (28) dans la partie d'insertion (21B).

5. Système médical (1) selon la revendication 1, comprenant en outre :
un inducteur (18B) relié en série à l'électrode de transmission d'énergie (18).
